# EUROPEAN PATENT APPLICATION

(11) **EP 2 693 215 A1**
(43) Date of publication of application: **05.02.2014**
(21) Application number: 12765825.0
(22) Date of filing: 23.03.2012
(51) Int. Cl.: G01N 33/66

(54) **BLOOD-GLUCOSE LEVEL MANAGEMENT SYSTEM**

(30) Priority: 31.03.2011 JP 2011078577
(71) Applicant: Terumo Kabushiki Kaisha, Tokyo 151-0072 (JP)
(72) Inventor: ASAMA Koichiro, Ashigarakami-gun Kanagawa 259-0151 (JP); TANZAWA Kazuhiko, Tokyo 103-0028 (JP)
(74) Representative: TBK
(86) International application number: PCT/JP2012/057581
(87) International publication number: WO 2012/133221

(57) **Abstract**

A blood-glucose level management system (101) is provided that uniformly collects blood-glucose level data from blood-glucose meters of a plurality of different specifications and that can manage it in a unified way.

A terminal (401) of the blood-glucose level management system (101) distinguishes a ward blood-glucose meter (103) and a personal blood-glucose meter (102) in the early stage of communication by inquiring a model name. Then, if the blood-glucose meter is the ward blood-glucose meter (103), blood-glucose level data is immediately downloaded and is uploaded to a server (104). If it is the personal blood-glucose meter (102), a patient ID or a serial number is acquired through a barcode reader (405) or an operation portion (412) and the patient ID is associated with the blood-glucose level data. The blood-glucose level management system (101) is configured as described above, and thus it is possible not only to handle the ward blood-glucose meter (103) and the personal blood-glucose meter (102) with the common system but also to uniformly manage the blood-glucose levels of all patients in a unified way.

## Description

### Technical Field

The present invention relates to a blood-glucose level management system, a blood-glucose level data transfer device, a blood-glucose level data transfer program and a blood-glucose level data transfer method.

More specifically, the present invention relates to a blood-glucose level management system that uniformly collects blood-glucose level data from blood-glucose meters of a plurality of different specifications and that can manage it in a unified way, and to a blood-glucose level data transfer device, a blood-glucose level data transfer program and a blood-glucose level data transfer method used therein.

### Background Art

As is known, diabetes is caused by abnormal insulin secretion of pancreas or decreased insulin sensitivity. In particular, for a diabetes patient of type 1 whose insulin secretion is stopped, it is necessary to measure a blood-glucose level before a meal and administer insulin according to the level.

Conventionally, in order for a patient himself/herself or his/her family member to easily measure a blood-glucose level at home, the applicant has developed, manufactured and sold a small-sized blood-glucose level measurement device (hereinafter a "blood-glucose meter") aimed at self-measurement. Moreover, the applicant is in the process of developing a blood-glucose meter that has various management functions applicable to a plurality of patients in a ward.

Note that, in the present specification, in order for these blood-glucose meters of different specifications to be distinguished, the former small-sized blood-glucose meter aimed at self-measurement is called a personal blood-glucose meter, and the latter blood-glucose meter for a ward is called a ward blood-glucose meter.

Note that, a patent application on the latter ward blood-glucose meter and a blood-glucose level management system filed by the applicant is described in Patent Literature 1.

### Citation List

### Patent Literature

PTL 1: International Publication WO2009/031636

### Summary of Invention

### Technical Problem

The applicant has so far established a blood-glucose level management system applicable to a personal blood-glucose meter as a personal computer application program and mainly provided it for hospitals. The hospitals utilize this blood-glucose level management system for both inpatients and outpatients.

Thereafter, the applicant has developed the ward blood-glucose meter disclosed in Patent Literature 1 and the blood-glucose level management system used therefor.

However, a large number of personal blood-glucose meters have already been widely used in hospitals, and it is difficult to replace them all in terms of cost. Hence, in hospitals, two blood-glucose level management systems, that is, the blood-glucose level management system of the personal blood-glucose meter and the blood-glucose level management system of the ward blood-glucose meter coexist, thus causing problems in the management and operation of blood-glucose level data.

The present invention is made in view of the forgoing, and has an object to provide a blood-glucose level management system that uniformly collects blood-glucose level data from blood-glucose meters of a plurality of different specifications and that can manage it in a unified way, a blood-glucose level data transfer device, a blood-glucose level data transfer program and a blood-glucose level data transfer method.

### Solution to Problem

To solve the above-described problems, according to the present invention, there is provided a blood-glucose level management system including: a first blood-glucose meter including: a blood-glucose level measurement portion that measures a blood-glucose level of a patient; and a first blood-glucose level small table having: a patient ID field in which a patient ID uniquely identifying the patient is stored; a blood-glucose level field in which the blood-glucose level of the patient measured with the blood-glucose level measurement portion is stored; and a measurement date and time field in which a measurement date and time when the blood-glucose level of the patient is measured with the blood-glucose level measurement portion is stored; a second blood-glucose meter including: the blood-glucose level measurement portion; and a second blood-glucose level small table having the blood-glucose level field and the measurement date and time field; a server including: a blood-glucose level table having the patient ID field, the blood-glucose level field, and the measurement date and time field; and a blood-glucose level data transfer device including: a communication portion that performs data communication with the first blood-glucose meter and the second blood-glucose meter; and an input and output control portion that is connected to the server through a network and is connected to the communication portion, that determines whether a blood-glucose meter in communication is the first blood-glucose meter or the second blood-glucose meter, that uploads details of the first blood-glucose level small table to the server when the blood-glucose meter is the first blood-glucose meter, and that identifies a patient ID for a blood-glucose level recorded in the second blood-glucose level small table when the blood-glucose meter is the second blood-glucose meter, and then uploads details of the second blood-glucose level small table together with the identified patient ID to the server.

The blood-glucose level data transfer device distinguishes the first blood-glucose meter and the second blood-glucose meter in the early stage of communication. Then, if the blood-glucose meter is the first blood-glucose meter, the details of the first blood-glucose level small table within the first blood-glucose meter are immediately uploaded to the server. If it is the second blood-glucose meter, the patient ID corresponding to the blood-glucose level recorded in the second blood-glucose level small table within the second blood-glucose meter is identified, and then the details of the second blood-glucose level small table are uploaded together with the identified patient ID to the server.

### Advantageous Effects of Invention

According to the present invention, it is possible to provide a blood-glucose level management system that uniformly collects blood-glucose level data from blood-glucose meters of a plurality of different specifications and that can manage it in a unified way, a blood-glucose level data transfer device, a blood-glucose level data transfer program and a blood-glucose level data transfer method.

### Brief Description of Drawings

Fig. 1 is a schematic diagram showing the overall configuration of a blood-glucose level management system that is an example of an embodiment of the present invention;
Fig. 2 is a functional block diagram of a ward blood-glucose meter;
Fig. 3 is a functional block diagram of a personal blood-glucose meter;
Fig. 4 is a functional block diagram of a terminal;
Fig. 5 is a functional block diagram of a server;
Fig. 6 is a field configuration diagram of various tables;
Fig. 7 is a flowchart showing the flow of a blood-glucose level data registration operation by the terminal;
Fig. 8 is a flowchart showing the flow of model identification processing by the terminal; and
Fig. 9 is a diagram showing an example of a blood-glucose level trend graph.

### Description of Embodiments

### [Overall configuration]

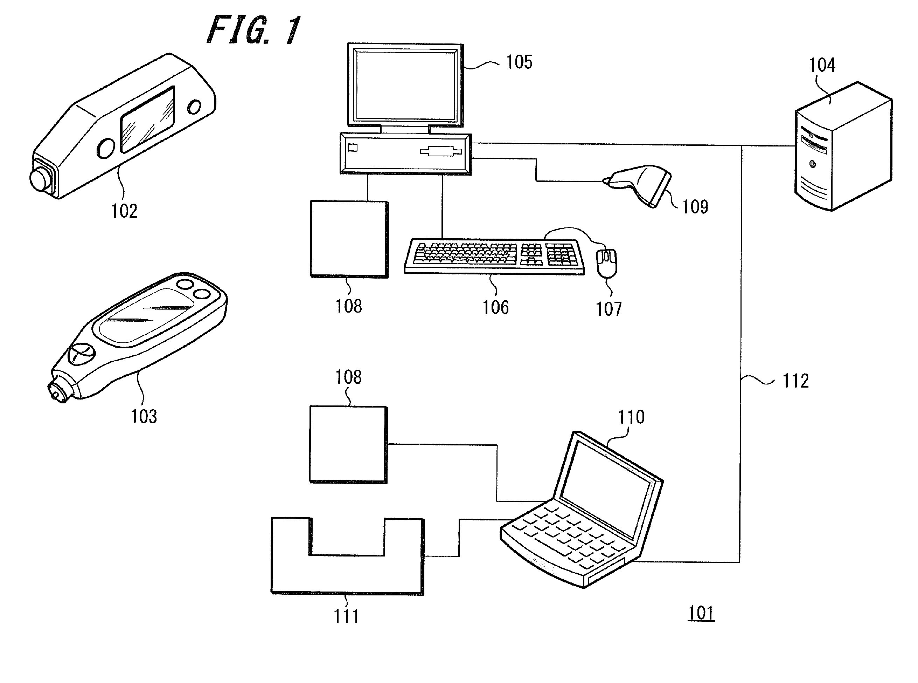

Fig. 1 is a schematic diagram showing the overall configuration of a blood-glucose level management system that is an example of the embodiment of the present invention.

The blood-glucose level management system 101 includes: a terminal 401 that receives blood-glucose level data from a personal blood-glucose meter 102 and a ward blood-glucose meter 103; and a server 104 that receives the blood-glucose level data from the terminal 401 and that records it in an internal database.

In Fig. 1, two terminals 401 are shown.

The first terminal 105 is configured by connecting an IC card reader 108 and a barcode reader 109 to a known personal computer to which a keyboard 106 and a mouse 107 are connected. The first terminal 105 mainly receives the blood-glucose level data from the personal blood-glucose meter 102 but can also be applied to the ward blood-glucose meter 103.

In the second terminal 110, an IC card reader 108 and a cradle 111 are connected to a known personal computer including a keyboard. The cradle 111 functions both as a charger for a lithium-ion battery incorporated in the ward blood-glucose meter 103 and as an interface for performing infrared communication with the ward blood-glucose meter 103. The second terminal 110 mainly receives the blood-glucose level data from the ward blood-glucose meter 103, and can also be applied to the personal blood-glucose meter 102 only for inpatients.

The keyboard 106, the mouse 107, the IC card reader 108 and the barcode reader 109 connected to the first terminal 105 and the IC card reader 108 and the cradle 111 connected to the second terminal 110 are connected to the terminal 401 through, for example, an general-purpose interface such as a USB.

The first terminal 105 and the second terminal 110 are operated by the same application program.

The IC card reader 108 is a near-field wireless communication interface; the specification of the IC card reader 108 is, for example, known Felica (registered trade mark).

An infrared communication function incorporated in the cradle 111 is, for example, known IrDA (Infrared Data Association).

Each of these communication interfaces transmits and receives data such as a blood-glucose level as stream data in a plain text format.

### [Ward blood-glucose meter 103]

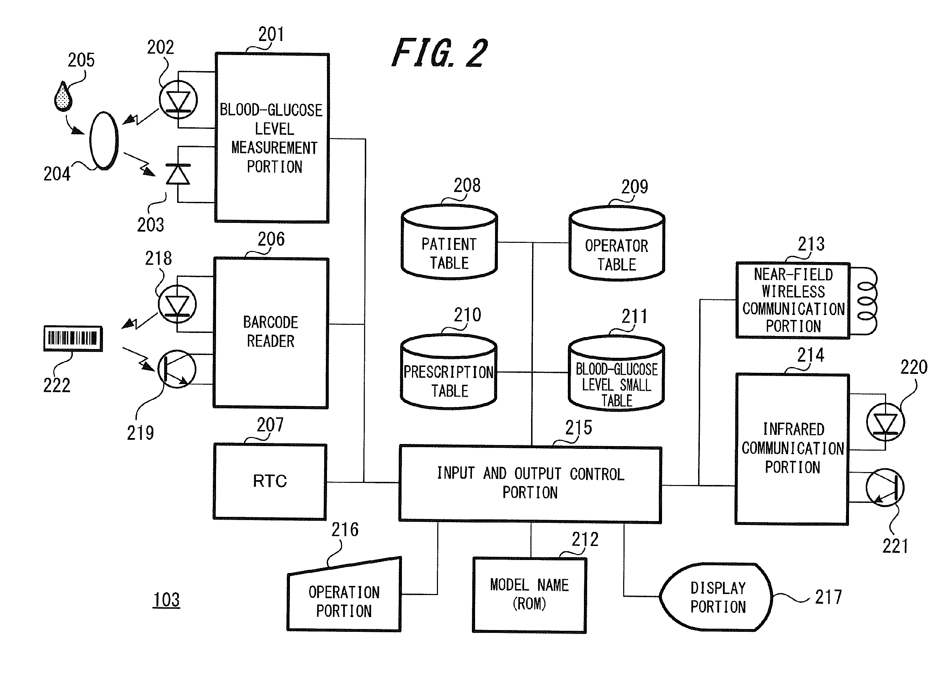

Fig. 2 is a functional block diagram of the ward blood-glucose meter 103. The ward blood-glucose meter 103, which can also be said to be a first blood-glucose meter, is an electronic device that is formed mainly with a microcomputer.

A blood-glucose level measurement portion 201 detects a variation in the optical reflectivity of test paper 204 whose color is changed by a blood 205 of a person to be measured, and measures a blood-glucose level.

The basic mechanism of blood-glucose measurement of the personal blood-glucose meter 102 and the ward blood-glucose meter 103 is the same as the conventional technology. The mechanism for measuring a blood-glucose level will be described briefly below.

The user of the ward blood-glucose meter 103 attaches an unillustrated measurement chip to the ward blood-glucose meter 103, and has the blood 205 of the person to be measured sucked into the measurement chip. This measurement chip incorporates the test paper 204 formed with a porous membrane such as polyether sulfone. Then, when the blood 205 sucked into the measurement chip reaches the test paper 204, the blood 205 reacts with a reagent contained in the test paper 204 to produce a color. Next, the ward blood-glucose meter 103 applies light emitted by an LED 202, which is a light emitting element, to the test paper 204, and receives the light reflected from the test paper 204 with a photodiode 203, which is a light receiving element. Then, after a predetermined reaction time has elapsed, the ward blood-glucose meter 103 converts an analog light reception intensity signal obtained from the light receiving element into a digital value, and thereafter converts this digital value into a blood-glucose level.

Note that, the mechanism on the side of the blood-glucose level meter is not limited to the above-described optical measurement system utilizing a color-producing reagent; a mechanism, such as an electrochemical sensor system, that can be conventionally used for the measurement of blood-glucose can be adopted.

A barcode reader 206 has the equivalent function as the barcode reader 109 connected to the first terminal 105 described above. The barcode reader 206 reads, for example, a barcode describing a patient ID printed on a name tag attached to a breast portion of the patient clothing of the patient and a barcode describing an operator ID printed on a name tag of a doctor or a nurse, and outputs text stream data encoded as the barcode.

A real time clock (hereinafter the "RTC") 207 is incorporated in many general microcomputers, and outputs date and time information.

Note that, the patient ID is a string of characters such as numerical values or alphabet that uniquely identifies (that is unique) the patient (the person to be measured). Likewise, the operator ID is a string of characters for uniquely identifying the operator of the blood-glucose meter.

A patient table 208 is table in which the patient IDs of patients and patient names are recorded.

An operator table 209 is a table in which the operator IDs of operators such as doctors and nurses and operator names are recorded.

A prescription table 210 is a table in which the patient IDs and the prescriptions of insulin and the like for the patients are recorded.

A blood-glucose level small table 211, which can also be said to be a first blood-glucose level small table, is a table in which the patient IDs, measured blood-glucose levels and the dates and times of the measurements are recorded.

A model name 212 is text data that shows the model name of the ward blood-glucose meter 103.

The patient table 208, the operator table 209, the prescription table 210, the blood-glucose level small table 211 and the model name 212 are stored in a nonvolatile storage such as an EEPROM. Although the model name 212 is set such that it cannot be rewritten and erased, the patient table 208, the operator table 209, the prescription table 210 and the blood-glucose level small table 211 are set such that they can be rewritten through the terminal 401.

A near-field wireless communication portion 213 is an interface that performs near-field wireless communication with the IC card reader 108 of the terminal 401 to transmit and receive data.

An infrared communication portion 214 is an interface that performs infrared communication with the cradle 111 of the terminal 401 to transmit and receive data.

The ward blood-glucose meter 103 can communicate with the terminal 401 using any of the communication functions of the near-field wireless communication portion 213 and the infrared communication portion 214.

An input and output control portion 215 controls the input and output of information. Specifically, the input and output control portion 215 reads the patient ID and the operator ID from the barcode reader 206 while displaying a screen indicating the operation on a display portion 217 according to operation information sent from an operation portion 216, then acquires blood-glucose level data from the blood-glucose level measurement portion 201, also acquires the date and time when the blood-glucose level data was acquired from the RTC 207, and associates these pieces of information with each other to record them in the blood-glucose level small table 211. Immediately after the blood-glucose level is measured, the input and output control portion 215 reads a prescription of insulin or the like from the prescription table 210 and displays it on the display portion 217 for a patient who requires the prescription. Then, the input and output control portion 215 performs bidirectional communication with the terminal 401 through the near-field wireless communication portion 213 or the infrared communication portion 214, and transmits information recorded in the blood-glucose level small table 211 to the terminal 401.

### [Personal blood-glucose meter 102]

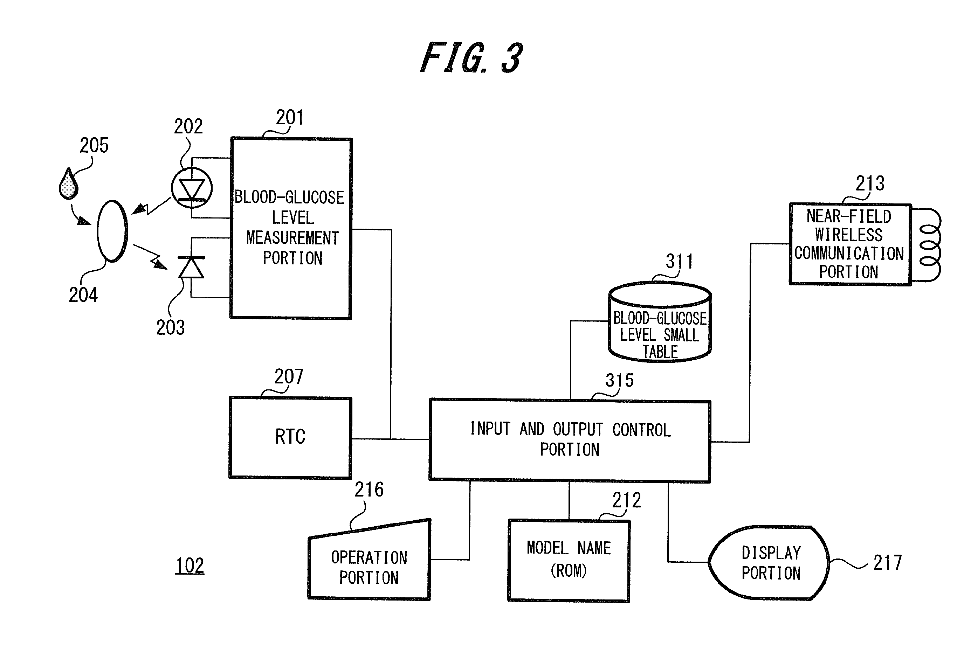

Fig. 3 is a functional block diagram of the personal blood-glucose meter 102. As with the ward blood-glucose meter 103, the personal blood-glucose meter 102, which can also be said to be a second blood-glucose meter, is an electronic device that is formed mainly with a microcomputer. Note that, in Fig. 3, the functional blocks whose functions are common to those in Fig. 2 are attached with like figure numbers, and their description will not be repeated.

As compared with the ward blood-glucose meter 103, in the personal blood-glucose meter 102, the barcode reader 206, the patient table 208, the operator table 209, the prescription table 210 and the infrared communication portion 214 are missing. Among these functional blocks, the functional blocks other than the infrared communication portion 214 are elements necessary for the ward blood-glucose meter 103 to mainly measure and record the blood-glucose levels of a plurality of patients.

Moreover, unlike the blood-glucose level small table 211 of the ward blood-glucose meter 103, a blood-glucose level small table 311, which can also be said to be a second blood-glucose level small table, does not include information on a plurality of patients and operators. Furthermore, unlike the input and output control portion 215 of the ward blood-glucose meter 103, an input and output control portion 315 does not have the function of dealing with information on a plurality of patients and operators or prescriptions.

Furthermore, since the personal blood-glucose meter 102 does not include the infrared communication portion 214, the communication with the terminal 401 is performed only by the near-field wireless communication portion 213.

On the other hand, the input and output control portion 315 has the function of storing, as history data, for example, up to 500 pieces of blood-glucose levels measured in the past.

### [Terminal 401]

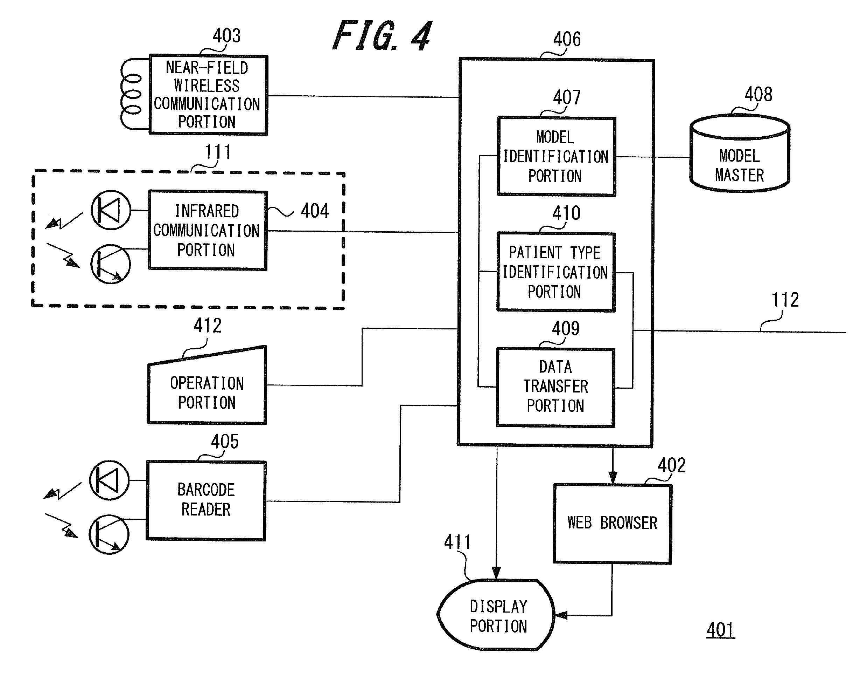

Fig. 4 is a functional block diagram of the terminal 401.

The terminal 401, which can also be said to be a blood-glucose level data transfer device and is a known personal computer, is operated by a known network OS. The personal computer reads and executes an application program for operating as the terminal 401 of the blood-glucose level management system 101 to communicate with the personal blood-glucose meter 102 and the ward blood-glucose meter 103 to receive (download) the blood-glucose level data and to communicate with the server 104 to transmit (upload) the blood-glucose level data to the server 104, and thereby operates as the terminal 401 of the blood-glucose level management system 101.

The network OS has the function of connecting to a known TCP/IP network, and is, for example, Windows (registered trademark). In addition to the network OS and the application program, a web browser 402 for displaying a graph indicating a blood-glucose level trend, which will be described later, is also installed in the terminal 401.

The IC card reader 108 is an entity of the near-field wireless communication portion 403, which is an interface for performing bidirectional communication with the near-field wireless communication portions 213 of the ward blood-glucose meter 103 and the personal blood-glucose meter 102 to download the blood-glucose level data.

An infrared communication portion 404 is incorporated in the cradle 111, and is an interface for performing bidirectional communication with the infrared communication portion 214 of the ward blood-glucose meter 103 to download the blood-glucose level data.

Since both the near-field wireless communication portion 403 and the infrared communication portion 404 perform bidirectional communication with the blood-glucose meter, these portions can be collectively referred to as a communication portion.

A barcode reader 405 is equivalent in terms of function to the barcode reader 206 incorporated in the ward blood-glucose meter 103 described above. The barcode reader 405 reads a barcode describing a patient ID printed on a name tag attached to a breast portion of the patient clothing of the patient and a barcode describing a serial number adhered to or printed on the housing of the personal blood-glucose meter 102, and outputs text stream data encoded as the barcode.

An input and output control portion 406 controls the input and output of information. Specifically, the input and output control portion 406 constantly monitors the near-field wireless communication portion 403, the infrared communication portion 404 and the operation portion 216, and first checks, if data is produced, with a model identification portion 407, the model of the blood-glucose meter against a model master 408 and specifies it. Next, if the model of the blood-glucose meter is the ward blood-glucose meter 103, the input and output control portion 406 immediately controls a data transfer portion 409, downloads all pieces of blood-glucose level data from the near-field wireless communication portion 403 or the infrared communication portion 404 and uploads them to the server 104. If the model of the blood-glucose meter is the personal blood-glucose meter 102, the input and output control portion 406 then acquires the patient ID or the serial number of the personal blood-glucose meter 102 from the barcode reader 405 or an operation portion 412, thereafter controls a patient type identification portion 410 to communicate with the server 104 and determines whether the patient is an inpatient or an outpatient. If the patient is an inpatient, the input and output control portion 406 controls the data transfer portion 409 to download the latest, one piece of blood-glucose level data from the near-field wireless communication portion 403 and uploads it to the server 104. If the patient is an outpatient, the input and output control portion 406 controls the data transfer portion 409 to download all pieces of blood-glucose level data from the near-field wireless communication portion 403, and uploads them to the server 104, and thereafter the input and output control portion 406 activates the web browser 402 and displays the blood-glucose level trend graph on a display portion 411 through the web browser 402.

The operation portion 412 whose entity is the keyboard 106 and the mouse 107 is used such as when the patient ID or the serial number is input instead of the barcode reader 405 and when the web browser 402 is operated.

Both the barcode reader 405 and the operation portion 412 can be collectively referred to as a patient ID input portion for inputting the patient ID or the serial number.

Fig. 4 shows all peripheral devices connected to the personal computer that is the terminal 401. However, if the application of the terminal 401 is specialized, some peripheral devices are not necessarily needed.

If the terminal 401 is specialized for the reception of the personal blood-glucose meter 102, the infrared communication portion 404 that is an interface dedicated for the ward blood-glucose meter 103 is not needed.

If the terminal 401 is specialized for the reception of the ward blood-glucose meter 103, the barcode reader 405 that identifies the patient ID to associate the blood-glucose level data of the personal blood-glucose meter 102 with the patient ID is not needed.

### [Server 104]

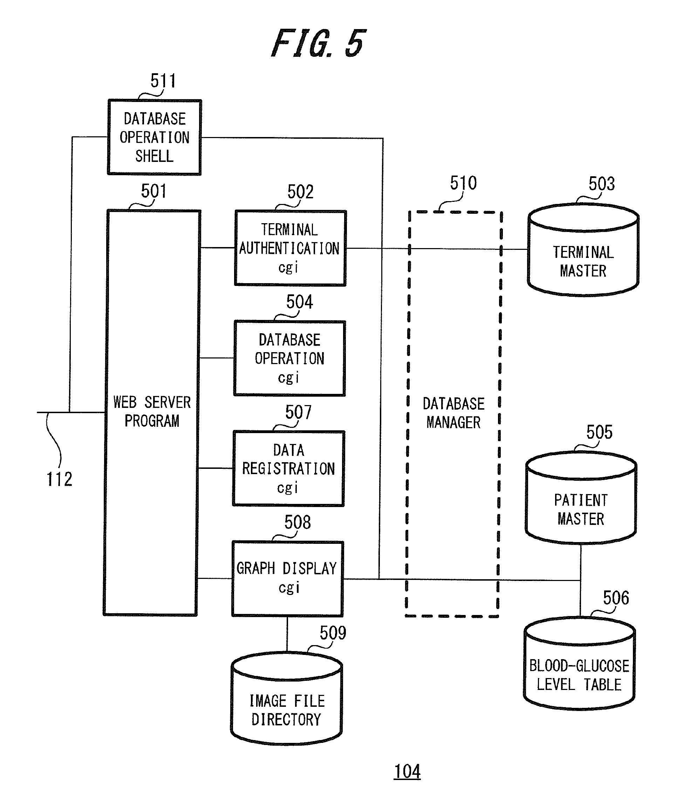

Fig. 5 is a functional block diagram of the server 104.

The server 104 functions both as a web server and as a database server.

A web server program 501 receives a request of the terminal 401, transmits an html (Hyper Text Markup Language) document file to the terminal 401 according to the request of the terminal 401, activates the cgi corresponding to the details of the request and transmits its results to the terminal 401.

A terminal authentication cgi 502 controls terminal authentication for determining the validity of the terminal 401 according to the request of the terminal 401. Hence, the terminal authentication cgi 502 exchanges login information with a terminal master 503.

A database operation cgi 504 performs, according to the request of the terminal 401, operations such as retrieval and the additional recording of a record on a patient master 505 and a blood-glucose level table 506.

A data registration cgi 507 additionally records, in the blood-glucose level table 506, the blood-glucose level data transmitted from the terminal 401.

A graph display cgi 508 retrieves, according to the request of the terminal 401, the blood-glucose level data on the patient specified from the blood-glucose level table 506, produces, based on the blood-glucose level data, image data on the blood-glucose level trend graph in an image file directory 509 and transmits it to the terminal 401.

The terminal master 503, the patient master 505 and the blood-glucose level table 506 can perform operations such as retrieval and registration through a database manager 510.

A database operation shell 511 is a command interpreter that uses the protocol of a web server program 501, that is, a port number different from http (TCP port number 80) and an application layer protocol to realize various operations on the terminal master 503, the patient master 505 and the blood-glucose level table 506.

In other words, communication means that operates the terminal master 503, the patient master 505 and the blood-glucose level table 506 can utilize not necessarily only http but various protocols.

### [Field configuration of a table]

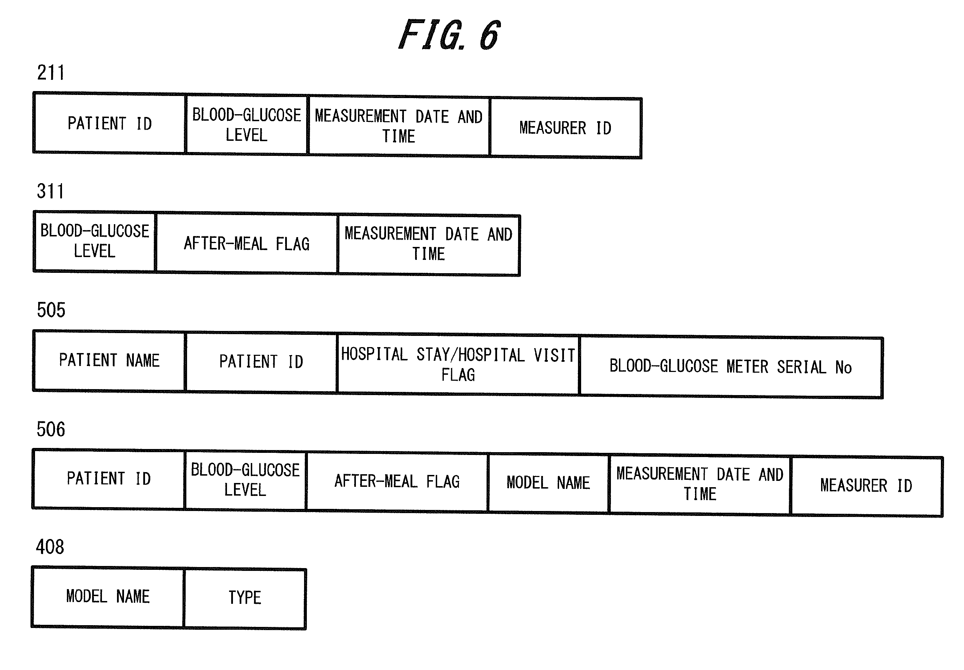

Fig. 6 is a field configuration diagram of various tables.

The blood-glucose level small table 211 of the ward blood-glucose meter 103 includes a patient ID field, a blood-glucose level field, a measurement date and time field and a measurer ID field.

The patient ID is stored in the patient ID field.

The blood-glucose level of the patient is stored in the blood-glucose level field.

The date and time when the blood-glucose level of the patient was measured is stored in the measurement date and time field.

The measurer ID of a person in charge such as a doctor or a nurse who has measured the blood-glucose level of the patient with the ward blood-glucose meter 103 is stored in the measurer ID field.

Since the ward blood-glucose meter 103 measures the blood-glucose levels of a plurality of patients, in the blood-glucose level small table 211, the patient ID field for distinguishing measured blood-glucose levels on a patient-by-patient basis is present.

The blood-glucose level small table 311 of the personal blood-glucose meter 102 includes a blood-glucose level field, an after-meal flag field and a measurement date and time field.

The blood-glucose level field and the measurement date and time field are the same as those in the blood-glucose level small table 211 of the ward blood-glucose meter 103.

A flag (hereinafter referred to as an "after-meal flag") indicating whether or not the blood-glucose level of the patient is measured after a meal with the personal blood-glucose meter 102 is stored in the after-meal flag field.

Since the personal blood-glucose meter 102 measures the blood-glucose level of one patient, unlike the blood-glucose level small table 211 of the ward blood-glucose meter 103, in the blood-glucose level small table 311, the patient ID field for distinguishing measured blood-glucose levels on a patient-by-patient basis is not present.

Note that, the blood-glucose level small table 311 can store up to 500 pieces of blood-glucose levels measured in the past and their associated data.

The patient master 505 of the server 104 includes a patient name field, a patient ID field, a hospital stay / hospital visit flag field and a blood-glucose meter serial number field.

The patient name is stored in the patient name field.

As with the patient ID field of the blood-glucose level small table 211 of the ward blood-glucose meter 103 and of the blood-glucose level small table 311 of the personal blood-glucose meter 102, the patient ID is stored in the patient ID field.

A flag indicating whether the patient is an inpatient or an outpatient is stored in the hospital stay / hospital visit flag field.

When the patient is an outpatient, the serial number of the personal blood-glucose meter 102 owned by the patient is stored in the blood-glucose meter serial number field.

Since the blood-glucose meter serial number is information that can be uniquely associated with the patient ID, the blood-glucose meter serial number can also be said to be patient ID associated information. Hence, the blood-glucose meter serial number field can also be said to be a patient ID associated information field.

The blood-glucose level table 506 of the server 104 includes a patient ID field, a blood-glucose level field, an after-meal flag field, a model name field, a measurement date and time field and a measurer ID field.

The patient ID field is the same as that of the patient master 505.

As with the blood-glucose level field of the blood-glucose level small table 211 of the ward blood-glucose meter 103 and of the blood-glucose level small table 311 of the personal blood-glucose meter 102, the blood-glucose level of the patient is stored in the blood-glucose level field.

The after-meal flag field is the same as that of the blood-glucose level small table 311 of the personal blood-glucose meter 102. Note that, in the case of the ward blood-glucose meter 103, the after-meal flag field is ignored.

The model name 212 of the blood-glucose meter that has measured the blood-glucose level of the patient is stored in the model name field.

The date and time when the blood-glucose level of the patient was measured is stored in the measurement date and time field.

The measurer ID of a person in charge such as a doctor or a nurse who has measured the blood-glucose level of the patient with the ward blood-glucose meter 103 is stored in the measurer ID field.

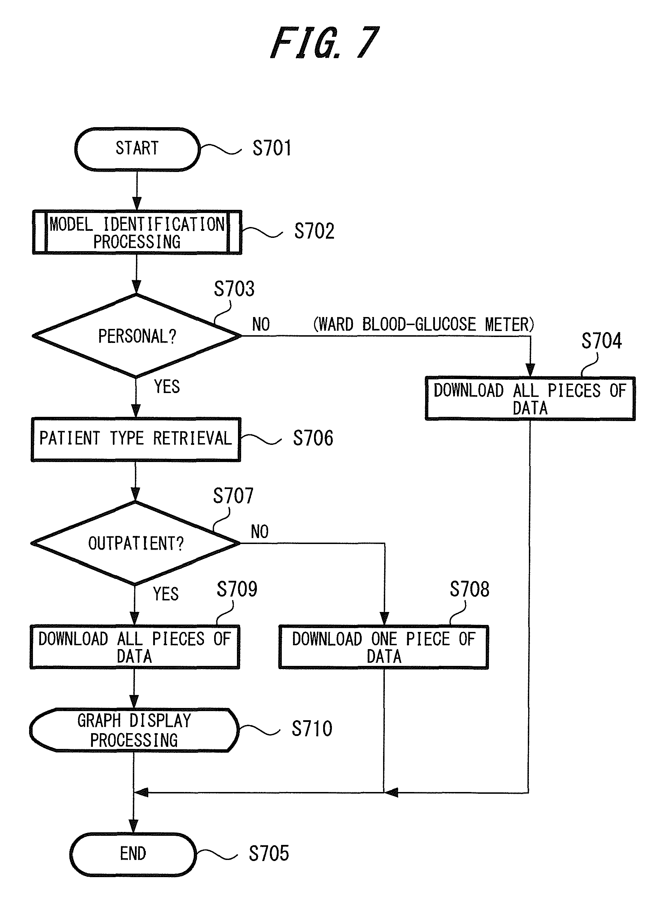

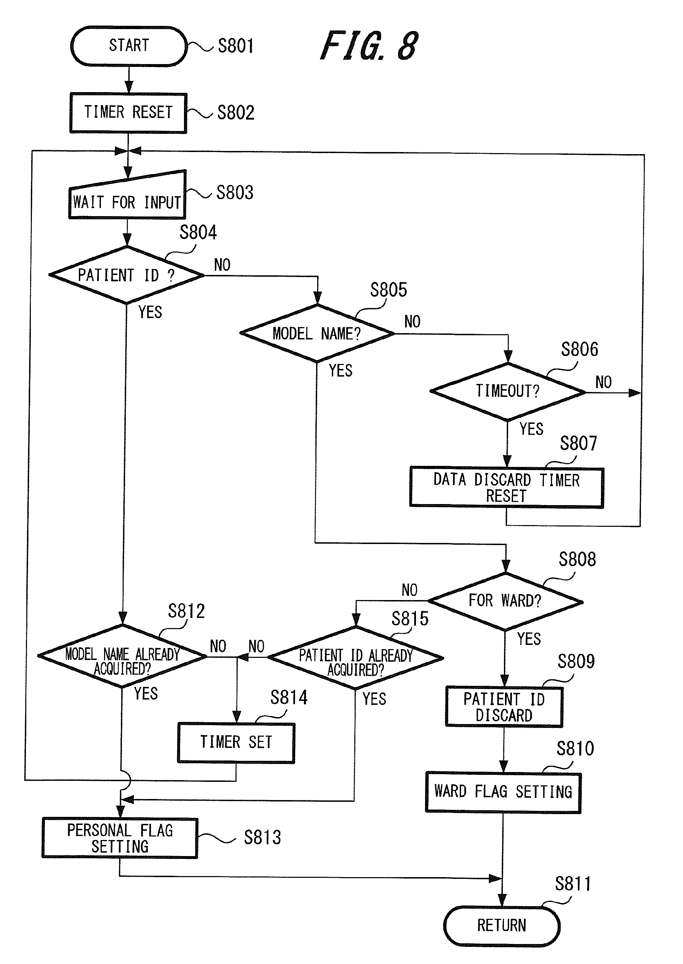

The blood-glucose level table 506 has a field configuration in which all data both in the blood-glucose level small table 211 of the ward blood-glucose meter 103 and in the blood-glucose level small table 311 of the personal blood-glucose meter 102 can be included. However, in the blood-glucose level small table 311 of the personal blood-glucose meter 102, the patient ID field is not present. Hence, when the blood-glucose level data is acquired from the personal blood-glucose meter 102, in order to associate the patient ID with the blood-glucose level data, the terminal 401 performs processing that identifies the patient ID. The details of the processing that acquires and identifies the patient ID will be described later with reference to the flowcharts of Figs. 7 and 8.

The model master 408 of the terminal 401 includes a model name field and a type field.

The model name field is the same as that of the blood-glucose level table 506.

A flag indicating whether the blood-glucose meter of the present model is the ward blood-glucose meter 103 or the personal blood-glucose meter 102 is stored in the type field.

Note that, although in the blood-glucose level management system 101 of the present embodiment, the model master 408 is included in the terminal 401, the model master 408 may be included in the server 104.

### [Operation]

The flow of the operation of the blood-glucose level management system 101 of the present embodiment will now be described with reference to the flowcharts of Figs. 7 and 8.

Fig. 7 is a flowchart showing the flow of a blood-glucose level data registration operation by the terminal 401.

When the process is started (S701), the input and output control portion 406 of the terminal 401 performs model identification processing (S702). In the model identification processing, the terminal 401 identifies the type of blood-glucose meter through the near-field wireless communication portion 403 (the IC card reader 108) or the infrared communication portion 404 (the cradle 111), and sets a flag that identifies the type. The details of the type identification processing will be described later with reference to Fig. 8.

When the type identification processing is completed, in an unillustrated RAM within the terminal 401, either a personal flag or a ward flag is set. Hence, the input and output control portion 406 of the terminal 401 then determines which of the personal flag and the ward flag is set, that is, which of the ward blood-glucose meter 103 and the personal blood-glucose meter 102 is currently performing communication (S703).

If as a result of the determination, the ward blood-glucose meter 103 is currently performing communication (NO in S703), the input and output control portion 406 of the terminal 401 downloads all pieces of blood-glucose level data from the ward blood-glucose meter 103 through the near-field wireless communication portion 403 or the infrared communication portion 404, transfers them to the server 104 (S704) and completes a series of processing steps (S705).

In step S703, if as a result of the determination, the personal blood-glucose meter 102 is currently performing communication (YES in S703), the input and output control portion 406 of the terminal 401 further inquires the patient ID or the serial number acquired in the model identification processing of the server 104 (S706) and determines whether the patient is an inpatient or an outpatient, that is, the details of the hospital stay / hospital visit flag of the patient master 505 (S707). Note that, in step S706, if the information acquired in the model identification processing is the serial number of the personal blood-glucose meter 102, the input and output control portion 406 of the terminal 401 acquires not only the hospital stay / hospital visit flag of the patient master 505 but also the patient ID from the server 104.

If as a result of the determination, the patient is an inpatient (NO in S707), the input and output control portion 406 downloads only the latest, one piece of blood-glucose level data from the personal blood-glucose meter 102, transfers it together with the patient ID to the server 104 (S708) and completes a series of processing steps (S705).

In step S707, if as a result of the determination, the patient is an outpatient (YES in S707), the input and output control portion 406 downloads all pieces of blood-glucose level data from the personal blood-glucose meter 102, and transfers them together with the patient ID to the server 104 (S709). Then, processing that displays the blood-glucose level trend graph of the patient is performed in concert with the server 104 (S710), and a series of processing steps are completed (S705).

Fig. 8 is a flowchart showing the flow of the model identification processing by the terminal 401. Fig. 8 is the details of step S702 in Fig. 7.

When the process is started (S801), the input and output control portion 406 first resets an unillustrated internal timer (S802). Then, the input and output control portion 406 waits for an input, for a short period of time, while monitoring the near-field wireless communication portion 403, the infrared communication portion 404, the barcode reader 405, the cradle 111 or the keyboard 106 (S803). The short period of time refers to, for example, a time of about 0.5 to 3 seconds. In the waiting time, if data is input from the near-field wireless communication portion 403, the barcode reader 405, the infrared communication portion 404 or the keyboard 106, the input and output control portion 406 immediately transitions to the subsequent processing. Moreover, also when the waiting time has elapsed, it transitions to the subsequent processing.

After the waiting for an input in step S803, the input and output control portion 406 determines whether or not the patient ID or the serial number has been acquired from the near-field wireless communication portion 403, the barcode reader 405 or the keyboard 106 (S804).

If the patient ID or the serial number could not be acquired (NO in S804), then the input and output control portion 406 determines whether or not the model name 212 has been acquired from the near-field wireless communication portion 403 or the infrared communication portion 404 (S805).

If the model name 212 could not be acquired (NO in S805), then the input and output control portion 406 checks the timer to determine whether or not a predetermined time has elapsed (S806). This predetermined time is, for example, about 30 seconds as an allowable range of time necessary for the personal blood-glucose meter 102 to acquire the patient ID and the model name 212.

If the predetermined time has not yet elapsed (NO in S806), the input and output control portion 406 returns again to step S803 and waits for an input.

If the predetermined time has elapsed (YES in S806), since the time is up, the input and output control portion 406 discards all data such as the patient ID, the serial number and the model name 212 that have already been acquired, resets the timer (S807), returns again to step S803 and waits for an input.

In step S805, if the acquired data is the model name 212 (YES in S805), then the input and output control portion 406 activates the model identification portion 407. The model identification portion 407 searches the model master 408 with the acquired model name 212, and determines whether the model is the ward blood-glucose meter 103 or the personal blood-glucose meter 102 (S808). If the model is the ward blood-glucose meter 103 (YES in S808), when the patient ID and the serial number have already been acquired, the input and output control portion 406 discards them (S809), sets the ward flag indicating that the blood-glucose meter is the ward blood-glucose meter 103 (S810) and completes a series of processing steps (S811).

In step S804, if the acquired data is the patient ID (YES in S804), then the input and output control portion 406 determines whether or not the model name 212 has already been acquired at this point (S812). If the model name 212 has already been acquired (YES in S812), the input and output control portion 406 sets the personal flag indicating that the blood-glucose meter is the personal blood-glucose meter 102 (S813) and completes a series of processing steps (S811).

In step S812, if the model name 212 has not been acquired yet (NO in S812), the input and output control portion 406 activates the timer to acquire the model name 212 (S814), returns again to step S803 and waits for an input.

Moreover, in step S808, if the blood-glucose meter is the personal blood-glucose meter 102 (NO in S808), then the input and output control portion 406 determines whether or not the patient ID has already been acquired (S815). If the patient ID has already been acquired (YES in S815), the input and output control portion 406 sets the personal flag indicating that the blood-glucose meter is the personal blood-glucose meter 102 (S813) and completes a series of processing steps (S811).

In step S815, if the patient ID has not been acquired yet (NO in S815), the input and output control portion 406 activates the timer to acquire the patient ID (S814), returns again to step S803 and waits for an input.

The input and output control portion 406 performs the following determination processing according to the flowchart of the model identification processing shown in Fig. 8.
- If as a result of information obtained by waiting for an input (S803), the blood-glucose meter in communication is the ward blood-glucose meter 103 (YES in S808), even when the patient ID and the serial number have previously been acquired with an erroneous operation or the like, the input and output control portion 406 discards them because they are not necessary (S809), and immediately sets the ward flag (S810).
- If as a result of information obtained by waiting for an input (S803), the blood-glucose meter in communication is the personal blood-glucose meter 102 (NO in S808), the input and output control portion 406 further determines whether or not the patient ID or the serial number has already been acquired at this point (S815). If the patient ID or the serial number has already been acquired (YES in S815), since the necessary information, that is, the personal blood-glucose meter 102 and the patient ID for association and information related to it are available, the input and output control portion 406 sets the personal flag (S813).

- If as a result of information obtained by waiting for an input (S803), the input information is the patient ID (YES in S804), the input and output control portion 406 further determines whether or not the model name 212 has already been acquired at this point (S812). If the model name 212 has already been acquired (YES in S812), since it is clear that the blood-glucose meter is the personal blood-glucose meter 102 (if the blood-glucose meter is the ward blood-glucose meter 103 (YES in S808), since the input and output control portion 406 does not return to step S803 in which it waits for an input), step S812 is not reached), the input and output control portion 406 sets the personal flag (S813).
- If as a result of information obtained by waiting for an input (S803), the input information is the patient ID (YES in S804), and the model name 212 has not been acquired yet (NO in S812), the input and output control portion 406 activates the timer to acquire the model name 212 (S814), and waits for an input again (S803).
- If as a result of information obtained by waiting for an input (S803), the blood-glucose meter is the personal blood-glucose meter 102 (NO in S808), and the patient ID or the serial number has not been acquired yet (NO in S815), the input and output control portion 406 activates the timer to acquire the patient ID or the serial number (S814) and waits for an input again (S803).

As described above, when the terminal 401 downloads the blood-glucose level data from the personal blood-glucose meter 102 and uploads it to the blood-glucose level table 506 of the server 104, it is necessary to acquire the patient ID that cannot be directly obtained from the personal blood-glucose meter 102 and associate it with the downloaded blood-glucose level data. If the model identification portion 407 determines that the blood-glucose meter is the personal blood-glucose meter 102, the input and output control portion 406 of the terminal 401 of the present embodiment acquires the patient ID or the serial number through the barcode reader 405 or the operation portion 412 and associates the patient ID with the blood-glucose level data.

Furthermore, if a target for which the blood-glucose level data is downloaded is the personal blood-glucose meter 102, the input and output control portion 406 of the terminal 401 determines, through the patient type identification portion 410, whether or not a target patient is an inpatient or an outpatient. Then, if the target patient is an outpatient, all pieces of blood-glucose level data included in the personal blood-glucose meter 102 are downloaded, and the blood-glucose level trend graph is displayed on the display portion 411 through the web browser 402. On the other hand, if the target patient is an inpatient, only the immediate and latest, one piece of blood-glucose level data included in the personal blood-glucose meter 102 is downloaded. In this case, the blood-glucose level trend graph is not displayed.

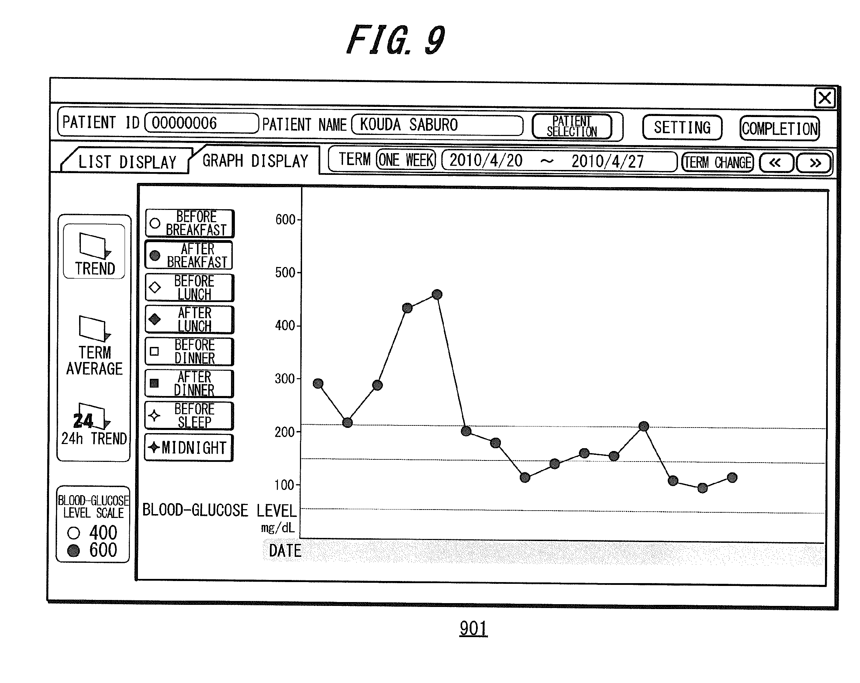

Fig. 9 is a diagram showing an example of the blood-glucose level trend graph.

The input and output control portion 406 of the terminal 401 passes, as an argument, a URL character string for access to the graph display cgi 508 of the server 104 to the web browser 402, and activates the web browser 402.

The web browser 402 accesses the graph display cgi 508 according to the URL character string passed as the argument.

The graph display cgi 508 identifies the patient ID from the immediate record that is additionally recorded in the blood-glucose level table 506 from the terminal ID of the terminal 401 which has been accessed, and produces image data on the blood-glucose level trend graph of the patient in the image file directory 509. The web browser 402 reads the image data on the blood-glucose level trend graph 901 produced in the image file directory 509, and displays it on the display portion 411.

In the blood-glucose level trend graph displayed on the display portion 411, the image of a line graph where the measurement date and time is on the horizontal axis and the blood-glucose level is on the vertical axis is displayed, and the name of the patient is also displayed.

The web browser 402 has a function as a graph display control portion for displaying the blood-glucose level trend graph 901 on the display portion 411.

The present embodiment has disclosed the blood-glucose level management system 101.

The terminal 401 of the blood-glucose level management system 101 distinguishes the ward blood-glucose meter 103 from the personal blood-glucose meter 102 by inquiring the model name in the early stage of communication. Then, if the blood-glucose meter is the ward blood-glucose meter 103, the blood-glucose level data is immediately downloaded and is uploaded to the server 104.

However, if it is the personal blood-glucose meter 102, when the blood-glucose level data is downloaded from the personal blood-glucose meter 102 and is uploaded to the blood-glucose level table 506 of the server 104, it is necessary to acquire the patient ID that cannot be directly obtained from the personal blood-glucose meter 102 and associate it with the downloaded blood-glucose level data. If the model identification portion 407 determines that the blood-glucose meter is the personal blood-glucose meter 102, the input and output control portion 406 of the terminal 401 of the present embodiment acquires the patient ID or the serial number through the barcode reader 405 or the operation portion 412, and associates the patient ID with the blood-glucose level data.

The blood-glucose level management system 101 is configured as described above, and thus it is possible not only to handle the ward blood-glucose meter 103 and the personal blood-glucose meter 102 with the common system but also to uniformly manage the blood-glucose levels of all patients in a unified way.

Furthermore, if a target for which the blood-glucose level data is downloaded is the personal blood-glucose meter 102, the input and output control portion 406 of the terminal 401 determines, through the patient type identification portion 410, whether or not a target patient is an inpatient or an outpatient. Then, if the target patient is an outpatient, all pieces of blood-glucose level data included in the personal blood-glucose meter 102 are downloaded, and the blood-glucose level trend graph is displayed on the display portion 411 through the web browser 402. On the other hand, if the target patient is an inpatient, only the immediate and latest, one piece of blood-glucose level data included in the personal blood-glucose meter 102 is downloaded. Additionally, in this case, the blood-glucose level trend graph is not displayed.

The blood-glucose level management system 101 is configured as described above, and thus it is possible to finely perform the operation even if the user of the personal blood-glucose meter 102 is different between an outpatient and an inpatient.

Although the example of the embodiment of the present invention has been described above, the present invention is not limited to the example of the embodiment described above, and other variations and applications are included as long as they do not depart from the spirit of the present invention described in the scope of claims.

### Reference Signs List

101: blood-glucose level management system, 102: personal blood-glucose meter, 103: ward blood-glucose meter, 104: server, 105: first terminal, 106: keyboard, 107: mouse, 108: IC card reader, 109: barcode reader, 110: second terminal, 111: cradle, 201: blood-glucose level measurement portion, 202: LED, 203: photodiode, 204: test paper, 205: blood, 206: barcode reader, 207: RTC, 208: patient table, 209: operator table, 210: prescription table, 211: blood-glucose level small table, 212: model name, 213: near-field wireless communication portion, 214: infrared communication portion, 215: input and output control portion, 216: operation portion, 217: display portion, 311: blood-glucose level small table, 315: input and output control portion, 401: terminal, 402: web browser, 403: near-field wireless communication portion, 404: infrared communication portion, 405: barcode reader, 406: input and output control portion, 407: model identification portion, 408: model master, 409: data transfer portion, 410: patient type identification portion, 411: display portion, 412: operation portion, 501: web server program, 502: terminal authentication cgi, 503: terminal master, 504: database operation cgi, 505: patient master, 506: blood-glucose level table, 507: data registration cgi, 508: graph display cgi, 509: image file directory, 510: database manager, 511: database operation shell, 901: blood-glucose level trend graph

## Claims

1. A blood-glucose level management system comprising:
a first blood-glucose meter including:
a blood-glucose level measurement portion that measures a blood-glucose level of a patient; and
a first blood-glucose level small table having: a patient ID field in which a patient ID uniquely identifying the patient is stored; a blood-glucose level field in which the blood-glucose level of the patient measured with the blood-glucose level measurement portion is stored; and a measurement date and time field in which a measurement date and time when the blood-glucose level of the patient is measured with the blood-glucose level measurement portion is stored;
a second blood-glucose meter including:
the blood-glucose level measurement portion; and
a second blood-glucose level small table having the blood-glucose level field and the measurement date and time field;
a server including:
a blood-glucose level table having the patient ID field, the blood-glucose level field, and the measurement date and time field; and
a blood-glucose level data transfer device including:
a communication portion that performs data communication with the first blood-glucose meter and the second blood-glucose meter; and
an input and output control portion that is connected to the server through a network and is connected to the communication portion, that determines whether a blood-glucose meter in communication is the first blood-glucose meter or the second blood-glucose meter, that uploads details of the first blood-glucose level small table to the server when the blood-glucose meter is the first blood-glucose meter, and that identifies a patient ID for a blood-glucose level recorded in the second blood-glucose level small table when the blood-glucose meter is the second blood-glucose meter, and then uploads details of the second blood-glucose level small table together with the identified patient ID to the server.

2. The blood-glucose level management system according to claim 1,
wherein the server further includes:
a patient master having: the patient ID field; a patient ID related information field in which patient ID related information associated with the patient ID is stored; and a hospital stay / hospital visit flag field in which a hospital stay / hospital visit flag indicating a type of patient whether the patient is an inpatient or an outpatient is stored,
wherein the blood-glucose level data transfer device includes:
a model identification portion that inquires of the blood-glucose meter in communication about a model name to determine whether the blood-glucose meter is the first blood-glucose meter or the second blood-glucose meter;
a patient ID input portion for acquiring the patient ID or the patient ID related information;
a patient type identification portion that acquires the patient ID or the patient ID related information from the patient ID input portion and thereafter acquires the hospital stay / hospital visit flag of the patient from the server when the model identification portion determines that the blood-glucose meter is the second blood-glucose meter; and
a data transfer portion that transfers the blood-glucose level data from the first blood-glucose meter and the second blood-glucose meter to the server, and
wherein when the patient type identification portion determines that the patient is an inpatient, the input and output control portion controls the data transfer portion to transfer only the latest, one piece of blood-glucose level data from the second blood-glucose meter to the server, whereas when the patient type identification portion determines that the patient is an outpatient, the input and output control portion controls the data transfer portion to transfer all pieces of blood-glucose level data from the second blood-glucose meter to the server.

3. The blood-glucose level management system according to claim 2,
wherein the blood-glucose level data transfer device further includes:
a display portion that displays a blood-glucose level trend graph; and
a graph display control portion that receives image data on the blood-glucose level trend graph from the server to display the blood-glucose level trend graph on the display portion, and
wherein when the patient type identification portion determines that the patient is an outpatient, the input and output control portion activates the graph display control portion, and causes the graph display control portion to receive the image data on the blood-glucose level trend graph from the server and to display the blood-glucose level trend graph on the display portion.

4. A blood-glucose level data transfer device comprising:
a communication portion that performs data communication with:
a first blood-glucose meter including: a blood-glucose level measurement portion that measures a blood-glucose level of a patient; and a first blood-glucose level small table having a patient ID field in which a patient ID uniquely identifying the patient is stored, a blood-glucose level field in which the blood-glucose level of the patient measured with the blood-glucose level measurement portion is stored and a measurement date and time field in which a measurement date and time when the blood-glucose level of the patient was measured with the blood-glucose level measurement portion is stored; and
a second blood-glucose meter including: the blood-glucose level measurement portion; and a second blood-glucose level small table having the blood-glucose level field and the measurement date and time field; and
an input and output control portion that is connected to a server through a network and is connected to the communication portion, that determines whether a blood-glucose meter in communication is the first blood-glucose meter or the second blood-glucose meter, that uploads details of the first blood-glucose level small table to the server when the blood-glucose meter is the first blood-glucose meter, and that identifies a patient ID for a blood-glucose level recorded in the second blood-glucose level small table when the blood-glucose meter is the second blood-glucose meter, and then uploads details of the second blood-glucose level small table together with the identified patient ID to the server.

5. A blood-glucose level data transfer program causing a computer to function as a blood-glucose level data transfer device including:
a communication portion that performs data communication with:
a first blood-glucose meter including: a blood-glucose level measurement portion that measures a blood-glucose level of a patient; and a first blood-glucose level small table having a patient ID field in which a patient ID uniquely identifying the patient is stored, a blood-glucose level field in which the blood-glucose level of the patient measured with the blood-glucose level measurement portion is stored and a measurement date and time field in which a measurement date and time when the blood-glucose level of the patient was measured with the blood-glucose level measurement portion is stored; and
a second blood-glucose meter including: the blood-glucose level measurement portion; and a second blood-glucose level small table having the blood-glucose level field and the measurement date and time field; and
an input and output control portion that is connected to a server through a network and is connected to the communication portion, that determines whether a blood-glucose meter in communication is the first blood-glucose meter or the second blood-glucose meter, that uploads details of the first blood-glucose level small table to the server when the blood-glucose meter is the first blood-glucose meter, and that identifies a patient ID for a blood-glucose level recorded in the second blood-glucose level small table when the blood-glucose meter is the second blood-glucose meter, and then uploads details of the second blood-glucose level small table together with the identified patient ID to the server.

6. A blood-glucose level data transfer method comprising:
a model name acquisition step of acquiring a model name from a blood-glucose meter:
a first blood-glucose level data transfer step of acquiring, when in the model name acquisition step, the blood-glucose meter is a ward blood-glucose meter including blood-glucose level data on a plurality of patients, all pieces of blood-glucose level data from the ward blood-glucose meter to transfer the blood-glucose level data to the server;
a patient ID acquisition step of acquiring a patient ID uniquely identifying the patient or information capable of identifying the patient ID; and
a second blood-glucose level data transfer step of acquiring, when in the model name acquisition step, the blood-glucose meter is a personal blood-glucose meter including blood-glucose level data on a single patient, the blood-glucose level data from the personal blood-glucose meter to transfer the blood-glucose level data together with the patient ID acquired in the patient ID acquisition step to the server.
